# DEMANDE DE BREVET EUROPEEN

(11) **EP 0 856 286 A1**
(43) Date de publication de la demande: **05.08.1998**
(21) Numéro de dépôt: 98400230.3
(22) Date de dépôt: 04.02.1998
(51) Int. Cl.: A61B 17/02

(54) **Ecarteur sternal**

(30) Priorité: 04.02.1997 FR 9701206
(71) Demandeur: Couetil, Jean-Paul, 75007 Paris (FR)
(72) Inventeur: Couetil, Jean-Paul, 75007 Paris (FR)
(74) Mandataire: Busnel, Jean-Benoît

(57) **Abrégé**

L'invention concerne un écarteur sternal du type comprenant un profilé central (1) équipé d'une crémaillère (10) et portant, d'une part, un bras mobile (2) pour le support de pinces (P) destinées au soulèvement du bord de l'un des hémi-sternums, et d'autre part, un bras fixe (3) avec un élément d'appui (A) sur le bord de l'autre hemi-sternum,
caractérisé en ce que lesdites pinces (P) comprennent une griffe d'accrochage (4) montée folle sur ledit bras mobile (2) et des moyens pour le verrouillage amovible et automatique d'une patte de contre-appui (5) sur ladite griffe (4) dans des positions angulaires relatives prédéterminées de façon à obtenir l'éversion du bord de l'hemi-sternum correspondant.

## Description

La présente invention concerne un écarteur sternal ou écarteur thoracique.

Ces écarteurs sont destinés à faciliter l'exposition des artères mammaires internes gauche et droite, appelées aussi artères thoraciques internes, pour permettre leur dissection chirurgicale aux fins d'utilisation comme greffon artériel lors des pontages coronariens.

Les artères mammaires sont situées anatomiquement à l'intérieur du thorax et leur trajet longe des deux bords latéraux du sternum à sa face profonde exactement en regard des cartilages costo-sternaux.

Après ouverture longitudinale et médiane du sternum, (voie classique et habituelle d'accès au coeur pour les opérations cardiaques), les artères mammaires se situent de part et d'autre de l'incision sternale et leur exposition aux fins de dissection nécessite un écartement antéro-postérieur des deux hémi-sternums.

Il existe déjà des écarteurs comme celui décrit dans le FR 2 599 238 qui comprennent un profilé central équipé d'une crémaillère et portant, d'une part, un bras mobile pour le support de pinces destinées au soulèvement du bord de l'un des hemi-sternums, et d'autre part, un bras fixe avec un élément d'appui sur le bord de l'autre hemi-sternum.

Cependant, la relativement faible longueur des griffes rend difficile le placement de celle-ci à la face profonde du sternum, en particulier, chez les patients obèses ou chez les patients dont la paroi thoracique est épaisse.

En outre, l'exposition des artères mammaires n'est obtenue que par l'écartement antéro-postérieur des deux hémi-sternums. Comme cet écarteur ne permet pas de replier ou d'éverser le bord latéral de l'hémi-sternum vers l'extérieur, pour obtenir une ouverture et une exposition suffisantes, il est nécessaire de procéder à un écartement important, susceptible d'occasionner des fractures des cartilages chondrocostaux dont la structure est fragile ou bien d'engendrer des douleurs post-opératoires.

De plus, un accroissement de l'écartement entraîne une compression des structures et organes sous-jacents à l'élément d'appui de l'écarteur, ce qui constitue une source de perturbation hémodynamique lors de la dissection de l'artère mammaire droite car l'élément d'appui placé sur l'hémi-sternum gauche refoule les structures cardiaques.

De plus, les difficultés d'exposition de l'artère mammaire droite sont accrues par la déformation thoracique rémanente créée pour la dissection de l'artère mammaire gauche qui a précédé le plus souvent celle de l'artère mammaire droite.

Dans cette situation, l'écarteur a tendance à s'horizontaliser, et l'artère mammaire droite est dès lors mal exposée, engendrant des difficultés chirurgicales de dissection.

Au surplus, l'inversion de l'écarteur pour son transfert du côté gauche au côté droit, ou inversement, est obtenue par des manoeuvres complexes de montage et démontage de l'appareil, sources de difficultés et de confusion.

En effet, l'adaptation de l'écarteur en vue d'effectuer la dissection de la mammaire controlatérale nécessite un démontage complet de toutes ses pièces et un remontage symétrique, ce qui rend la manipulation longue et fastidieuse.

La présente invention a pour but de résoudre les problèmes techniques posés par l'art antérieur de manière simple et efficace.

Ce but est atteint, conformément à l'invention au moyen d'un écarteur sternal du type comprenant un profilé central équipé d'une crémaillère et portant, d'une part, un bras mobile pour le support de pinces destinées au soulèvement du bord de l'un des hémi-sternums, et d'autre part, un bras fixe avec un élément d'appui sur le bord de l'autre hemi-sternum,
caractérisé en ce que lesdites pinces comprennent une griffe d'accrochage montée folle sur ledit bras mobile et des moyens pour le verrouillage amovible et automatique d'une patte de contre-appui sur ladite griffe dans des positions angulaires relatives prédéterminées de façon à obtenir l'éversion du bord de l'hemi-sternum correspondant.

Selon une caractéristique avantageuse, ledit profilé central, ledit bras mobile et ledit bras fixe s'étendent dans un même plan.

Selon un mode de réalisation particulier, ladite patte de contre-appui a un profil cintré selon son axe longitudinal en définissant une forme en T.

Selon une autre caractéristique, ladite griffe est portée par une bague de raccordement avec ledit bras mobile.

Selon encore une autre caractéristique, ladite patte de contre-appui est portée par un étrier dont chacune des branches en regard est percée d'un orifice destiné à recevoir librement ledit bras mobile.

Selon une variante, ladite bague est logée dans ledit étrier.

Selon un autre mode de réalisation, lesdits moyens pour le verrouillage amovible de la patte de contre-appui sur ladite griffe sont constitués d'une languette élastique dont la face inférieure de ladite languette est fixée sur un ressort solidaire de la patte et dont une extrémité est susceptible de se bloquer dans des crans ménagés sur ladite griffe.

De préférence, l'autre extrémité de la languette est libre et est disposée à distance de la patte formant butée.

Selon une autre caractéristique, ledit bras mobile est solidaire d'un curseur engrenant, d'une part, ladite crémaillère et entraîné, d'autre part, au moyen d'une manivelle escamotable et amovible.

Un moyeu de transmission fait saillie de part et d'autre du curseur en portant deux alésages symétriques pour le montage de la manivelle.

Selon encore une autre caractéristique, ledit élément d'appui, solidaire du bras fixe, est constitué de deux lames perpendiculaires définissant une section en V.

De préférence, chacune desdites lames est inclinée avec un angle de 45º par rapport au plan de la crémaillère.

Selon une caractéristique spécifique, ladite crémaillère est réalisée en creux dans ledit profilé central.

L'écarteur de l'invention permet d'obtenir une exposition optimale des deux artères mammaires en éloignant de façon atraumatique les deux hémi-sternums.

En effet, après soulèvement puis éversion de l'hémi-sternum, l'artère mammaire apparaît plus distinctement dans le champ visuel de l'opérateur et ceci conjointement à une présentation améliorée de la face profonde de l'hémi-sternum, ce qui facilite grandement la dissection de l'artère.

L'amplitude de l'écartement antéro-postérieur des deux parties du sternum peut donc être réduite tout en conservant une exposition optimale des artères mammaires, ce qui diminue d'autant la compression des organes sous-jacents à l'élément d'appui reposant sur l'hémi-sternum controlatéral et élimine le risque de fracture chondrocostale.

En outre, le risque de perturbation hémodynamique lors de la dissection de l'artère mammaire droite, consécutive à la compression des structures cardiaques est évité. Grâce au réglage aisé de la position de la patte de contre-appui sur la griffe de soulèvement, l'amplitude de l'éversion sternale peut être ajustée, ce qui permet d'éviter tout traumatisme pariétal en respectant le caractère souple ou rigide des cartilages costaux selon les patients. L'élément d'appui reposant sur l'hémi-sternum opposé à celui où est effectuée la dissection mammaire est très allongé, ce qui répartit au mieux les forces de compression et diminue d'autant l'effet d'enfoncement du sternum contribuant ainsi à protéger les organes sous-jacents.

L'intégration des dents de la crémaillère à l'intérieur du corps du profilé central élimine le risque d'incarcération des tissus cutanés et sous-cutanés lors du déplacement du curseur.

Enfin, les manoeuvres d'inversion de l'écarteur qui permettent de passer de la configuration adaptée à la dissection de l'artère gauche à celle pour disséquer l'artère droite, et vice-versa, ne nécessitent plus de démontage complexe et fastidieux de l'ensemble des pièces de l'écarteur. Ces manoeuvres s'effectuent désormais simplement en inversant les deux griffes et en permutant la manivelle amovible sur la partie opposée du moyeu du curseur.

Cette disposition confère à l'écarteur de l'invention une grande souplesse d'utilisation.

La patte de contre-appui, tout en permettant l'éversion adaptable et réglable de l'hémi-sternum après élévation, permet de maintenir l'orientation du plan de l'écarteur à 45º et l'empêche ainsi de prendre une position horizontale notamment lors de la dissection de la seconde artère mammaire, du fait de la déformation rémanente du thorax engendrée par la dissection de la première artère.

L'invention sera mieux comprise à la lecture de la description qui va suivre, accompagnée des dessins sur lesquels :
- la figure 1 représente une vue en perspective d'un mode de réalisation de l'écarteur de l'invention ;
- la figure 2 représente une vue de profil de l'écarteur de la figure 1;
- la figure 3 représente une vue en perspective d'une patte de contre-appui ;
- la figure 4 représente une vue de profil en coupe partielle d'une griffe de soulèvement ;
- la figure 5 représente une vue de dos de la griffe de la figure 4 ;
- la figure 6 représente une vue en coupe partielle et de profil de la griffe de la figure 4.

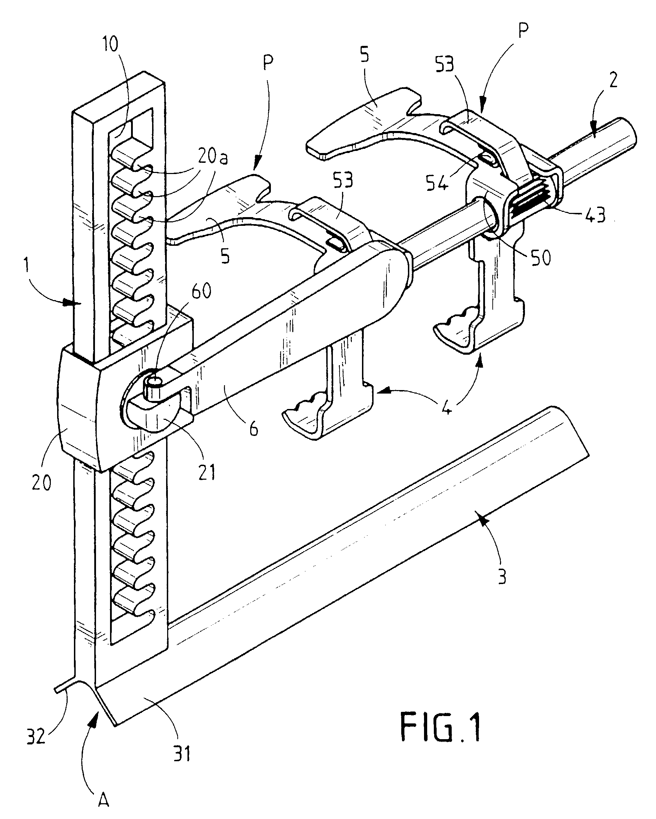

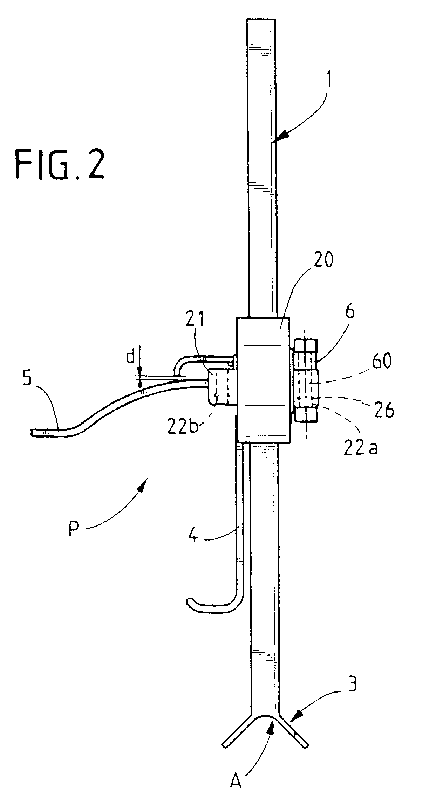

L'appareil représenté sur les figures 1 et 2 est un écarteur thoracique permettant d'effectuer, à des fins chirurgicales, l'écartement des deux hémi-sternum résultant de l'incision médiane du thorax en vue de procéder à une dissection des artères mammaires droite et gauche.

Cet écarteur comprend un profilé central 1 équipé d'une crémaillère 10 et portant un bras mobile 2 pour le support de deux pinces P destinées au soulèvement du bord latéral de l'un des hémi-sternums.

La position du bras 2 est donc réglable sur le profilé 1.

Le profilé central 1 porte également un bras fixe 3 avec un élément A assurant l'appui sur le bord latéral de l'autre hémi-sternum situé en regard.

Chaque pince P comprend une griffe d'accrochage 4 montée folle sur le bras mobile 2 ainsi qu'une patte de contre-appui 5. Les pinces comprennent également des moyens pour assurer la fixation et le verrouillage amovibles et automatiques de la patte de contre-appui 5 sur ladite griffe 4 dans des positions angulaires relatives prédéterminées. La griffe 4 vient s'accrocher à l'intérieur du thorax sous le bord latéral de l'hémi-sternum correspondant tandis que la patte 5 vient en appui sur la face externe du thorax, à distance de ce même bord latéral.

Le rapprochement des pattes 5 et des griffes 4 en pivotement autour du bras 2 entraîne l'éversion du bord latéral de l'hémi-sternum, c'est-à-dire son repli vers l'extérieur.

Le verrouillage de la patte 5 sur le griffe 4 est obtenu automatiquement quand la distance angulaire qui les sépare est minimum et quand les efforts d'action et de réaction exercés sur les bords latéraux de l'hémi-sternum et sur l'écarteur sont équilibrés. Cet équilibre dépend notamment, pour chaque patient, de la position donnée au bras mobile 2 par rapport au profilé central 1 via la crémaillère 10.

Le bras mobile 2 est solidaire d'un curseur 20 entourant le profilé central 1. Le curseur 20 engrène, d'une part, les dents 20a de la crémaillère 10 et est entraîné en translation, d'autre part, au moyen d'une manivelle 6 escamotable montée de manière amovible sur un moyeu de transmission 21. Le moyeu 21 fait saillie de part et d'autre du curseur 20 et comporte deux alésages symétriques 22a,22b adaptés et destinés à recevoir un doigt 60 solidaire de l'extrémité de la manivelle 6 et assurant la laison entre cette dernière et le moyeu.

Le doigt 60 se verrouille dans chaque alésage 22a,22b au moyen d'un anneau 26 qui permet également le pivotement de la manivelle 6 entre une position d'escamotage où elle s'étend parallèlement au plan de l'écarteur avec un encombrement minimum et une position de travail où elle s'étend à 90º de ce plan.

L'inversion de l'écarteur s'effectue notamment en libérant la manivelle 6 du curseur 20 par dégagement du doigt 60 de l'un des alésages et verrouillage dans l'alésage symétrique.

le profilé central 1, le bras fixe 3 et le bras mobile 2 sont coplanaires quelle que soit la position du curseur 20 sur la crémaillère 10.

La crémaillère 10 est réalisée en creux dans le profilé central 1 pour éviter tout traumatisme de la plaie opératoire à son contact.

L'élément d'appui A solidaire du bras fixe 3 est constitué de deux lames 31, 32 perpendiculaires entre elles et définissant sur le bras 3 une section en V.

Chacune des lames 31, 32 est ici inclinée avec un angle de 45º par rapport au plan de la crémaillère 10.

La gorge, ouverte vers l'extérieur et délimitée par les deux lames 31,32, est destinée à recevoir le bord latéral de l'hémi-sternum symétrique de celui qui est engagé dans les pinces P.

Toutefois, seule la lame qui se trouve à l'extérieur du thorax est en appui sur ce bord latéral car l'autre lame s'étend alors en saillie à l'intérieur, à 90º, en formant une face d'arrêt pour le bord de l'hémi-sternum.

Dans le mode de réalisation représenté, l'élément d'appui A est réalisé sur toute la longueur du bras 3.

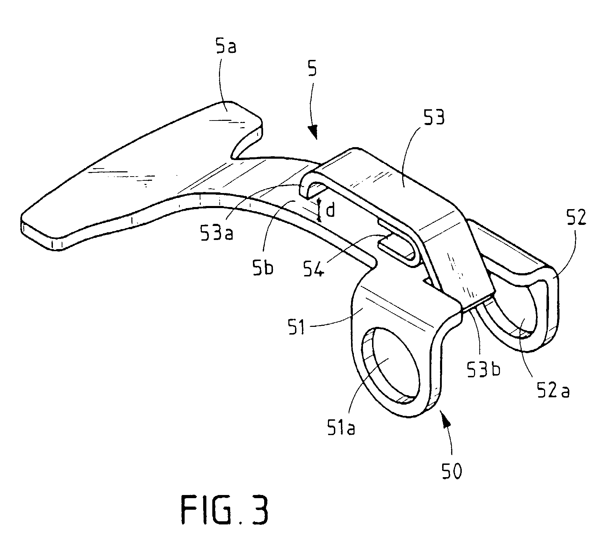

La figure 3 représente un mode de réalisation d'une patte de contre-appui 5.

La patte 5 est portée par un étrier 50 dont chacune des branches en regard 51,52 est percée d'un orifice 51a, 52a destiné à recevoir librement le bras mobile 2 qui traverse ainsi l'étrier 50 de part en part.

La patte 5 a un profil cintré selon son axe longitudinal en définissant une forme générale en T.

La zone avant plane 5a de la patte 5 qui exerce les efforts d'appui externes, s'étend donc transversalement à la zone intermédiaire cintrée 5b assurant la liaison avec l'étrier 50.

De la longueur de la zone intermédiaire 5b dépend l'amplitude de l'éversion.

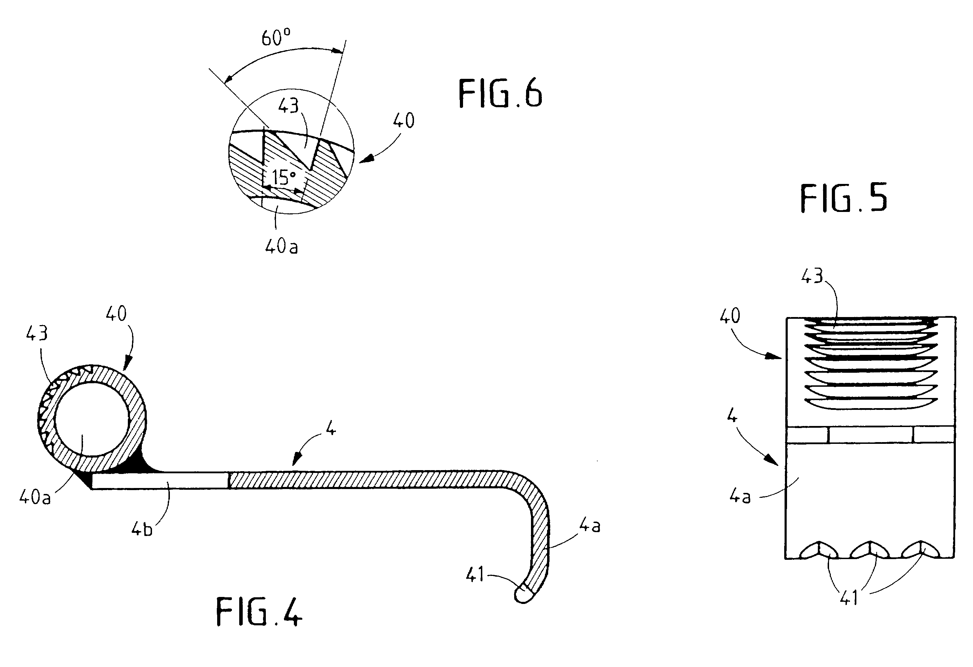

Les figures 4 et 5 représentent un mode de réalisation d'une griffe 4.

La griffe 4 est portée au niveau de son extrémité 4b par une bague 40 de raccordement avec le bras mobile 2.

La griffe 4 a une extrémité recourbée 4a pourvue de dents 41 destinées à s'accrocher sur la paroi interne de l'hémi-sternum.

La bague 40 est destinée à être logée dans l'étrier 50 et possède un orifice traversant 40a destiné à recevoir librement le bras mobile 2, formant alors l'axe du pivotement de la patte 5 sur la griffe 4 à la manière d'une charnière.

Les moyens destinés à assurer le verrouillage amovible et automatique de la patte 5 sur la griffe 4 dans différentes positions angulaires, sont constitués d'une languette élastique 53 montée sur la face extérieure de la patte 5.

L'extrémité 53a de la languette est libre et est disposée à une distance d de la zone de liaison 5b de la patte 5 formant butée, tandis que l'autre extrémité 53b, qui est libre également, fait saillie à l'intérieur de l'étrier 50 pour venir se bloquer dans des crans 43 ménagés sur la face latérale de la bague 40.

La face interne de la languette 53 est fixée sur un ressort 54 en forme de boucle ouverte solidaire de la zone de liaison 5b de la patte 5.

Le bord libre de l'extrémité 53b de la languette 53 est de préférence biseauté pour se conformer au profil des crans 43 et parfaire le verrouillage.

Les crans 43 représentés sur la figure 6 ont une section formée d'une face inclinée 43a avec un angle voisin de 60º par rapport au rayon de la bague 40 et d'une face radiale 43b en regard.

La longueur angulaire des crans 43 est d'environ 15º.

Lorsque l'opérateur déplace le curseur 20 sur la crémaillère 10 au moyen de la manivelle 6 pour éloigner le bras mobile 2 du bras fixe 3, les pinces P et l'élément d'appui A qui sont respectivement en prise sur chaque hémi-sternum, les forcent à s'écarter dans le plan à 45º défini par le profilé central 1, le bras mobile 2 et le bras fixe 3.

Au cours de ce déplacement, le bord biseauté de l'extrémité 53b glisse sur la face inclinée 43a des crans 43 en provoquant le basculement élastique de la languette 53 sur le ressort 54. Au cours de cette phase, la zone de laison 5b sert de butée en limitant le basculement vers l'avant de la languette 53. Puis le bord biseauté de l'extrémité 53b vient se bloquer automatiquement en butée contre la face radiale 43b du cran 43 adjacent.

Conjointement, chaque pince P pivote autour de l'axe formé par le bras mobile 2 pour s'orienter vers le thorax, ce qui, compte tenu de l'appui externe de la patte 5 à distance du bord de l'hémi-sternum, a pour effet de l'éverser par une déformation assimilable à une torsion en exposant ainsi l'artère mammaire sous-jacente.

## Revendications

1. Ecarteur sternal du type comprenant un profilé central (1) équipé d'une crémaillère (10) et portant, d'une part, un bras mobile (2) pour le support de pinces (P) destinées au soulèvement du bord de l'un des hémi-sternums, et d'autre part, un bras fixe (3) avec un élément d'appui (A) sur le bord de l'autre hemi-sternum,
caractérisé en ce que lesdites pinces (P) comprennent une griffe d'accrochage (4) montée folle sur ledit bras mobile (2) et des moyens pour le verrouillage amovible et automatique d'une patte de contre-appui (5) sur ladite griffe (4) dans des positions angulaires relatives prédéterminées de façon à obtenir l'éversion du bord de l'hemi-sternum correspondant.

2. Ecarteur selon la revendication 1, caractérisé en ce que ledit profilé central (1), ledit bras mobile (2) et ledit bras fixe (3) s'étendent dans un même plan.

3. Ecarteur selon la revendication 1 ou 2, caractérisé en ce que ladite patte (5) de contre-appui a un profil cintré selon son axe longitudinal en définissant une forme en T.

4. Ecarteur selon l'une des revendications précédentes, caractérisé en ce que ladite griffe (4) est portée par une bague (40) de raccordement avec ledit bras mobile (2).

5. Ecarteur selon l'une des revendications précédentes, caractérisé en ce que ladite patte (5) de contre-appui est portée par un étrier (50) dont chacune des branches en regard (51,52) est percée d'un orifice (51a, 52a) destiné à recevoir librement ledit bras mobile (2).

6. Ecarteur selon les revendications 4 et 5, caractérisé en ce que ladite bague (40) est logée dans ledit étrier (50).

7. Ecarteur selon l'une des revendications précédentes, caractérisé en ce que lesdits moyens pour le verrouillage amovible de la patte (5) de contre-appui sur ladite griffe (4) sont constitués d'une languette élastique (53) dont la face inférieure de ladite languette (53) est fixée sur un ressort (54) solidaire de la patte (5) et dont une extrémité (53b) est susceptible de se bloquer dans des crans ménagés sur ladite griffe (4).

8. Ecarteur selon la revendication 7, caractérisé en ce que l'autre extrémité (53a) de la languette (53) est libre et est disposée à distance de la patte (5) formant butée.

9. Ecarteur selon l'une des revendications précédentes, caractérisé en ce que ledit bras mobile (2) est solidaire d'un curseur (20) engrenant, d'une part, ladite crémaillère (10) et entraîné, d'autre part, au moyen d'une manivelle (6) escamotable et amovible.

10. Ecarteur selon la revendication 9, caractérisé en ce qu'un moyeu de transmission (21) fait saillie de part et d'autre du curseur (20) en portant deux alésages symétriques (22a,22b) pour le montage de la manivelle (6).

11. Ecarteur selon l'une des revendications précédentes, caractérisé en ce que ledit élément d'appui (A) solidaire du bras fixe (3) est constitué de deux lames perpendiculaires (31,32) définissant une section en V.

12. Ecarteur selon la revendication 11, caractérisé en ce que chacune desdites lames (31,32) est inclinée avec un angle de 45º par rapport au plan de la crémaillère (10).

13. Ecarteur selon l'une des revendications précédentes, caractérisé en ce que ladite crémaillère (10) est réalisée en creux dans ledit profilé central (1).
